# EUROPÄISCHE PATENTSCHRIFT

(11) **EP 1 030 650 B1**
(45) Veröffentlichungstag und Bekanntmachung des Hinweises auf die Patenterteilung: **13.02.2002**
(21) Anmeldenummer: 98965147.6
(22) Anmeldetag: 31.10.1998
(51) Int. Cl.: A61K 7/42

(54) **STABILE KOSMETISCHE FORMULIERUNG ENTHALTEND BUTYLMETHOXYDIBENZOYLMETHAN**
STABLE COSMETIC FORMULATION CONTAINING BUTYLMETHOXYDIBENZOYLMETHANE
FORMULATION COSMETIQUE STABLE CONTENANT DU BUTYLMETHOXYDIBENZOYLMETHANE

(30) Priorität: 12.11.1997 DE 19750029
(43) Veröffentlichungstag der Anmeldung: 30.08.2000
(73) Patentinhaber: MERCK PATENT GmbH, 64293 Darmstadt (DE)
(72) Erfinder: KURZ, Thekla, D-64285 Darmstadt (DE); DRILLER, Hansjürgen, D-64853 Otzberg (DE); HITZEL, Sabine, D-64409 Messel (DE); WILLE, Dorothee, D-64291 Darmstadt (DE)
(86) Internationale Anmeldenummer: EP9806901
(87) Internationale Veröffentlichungsnummer: WO9924006

(56) Entgegenhaltungen:
- WO-A-92/17159
- WO-A-94/21221
- WO-A-97/47280
- WO-A-98/13016
- DATABASE WPI Week 9642 Derwent Publications Ltd., London, GB; AN 96-421855 XP002099504 & JP 08 208403 A (KANEBO), 13. August 1996

## Beschreibung

Die vorliegende Erfindung betrifft unter anderem die Verwendung von Aldehyden und/oder Ketonen zur Verbesserung der Stabilität von kosmetischen Formulierungen, die als organischen Lichtschutzfilter Butylmethoxydibenzoylmethan kombiniert mit anorganischen Lichtschutzfiltem enthalten.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten. Im extremen Fall kommt es bei manchen Menschen zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Die heute üblichen Sonnenschutzfilter in der Kosmetik werden in UVA- bzw. UVB-Filter unterteilt. Für beide UV-Bereiche gibt es viele aus der Fachliteratur bekannte und bewährte Substanzen, beispielsweise seien hier nur Substanzen wie Eusolex ® 6300 oder Eusolex® 232, Dibenzoylmethane wie Eusolex® 9020 (Butylmethoxydibenzoylmethan als UVA-Filter) oder Eusolex® 8020, Benzophenone oder auch Octyl Triazone (Uvinul® T 150) aufgeführt.

Das Problem bei vielen Sonnenschutzpräparaten ist jedoch, daß viele Lichtschutzfilter empfindliche Substanzen sind und eine geringe Stabilität gegenüber den ultravioletten Strahlen aufweisen; sie zersetzen sich mehr oder weniger schnell. Dadurch ergeben sich Probleme bei der Haltbarkeit bzw. Wirksamkeit der entsprechenden kosmetischen Formulierungen.

in Sonnenschutzformulierungen werden auch organische Lichtschutzfilter mit anorganischen Filtern wie Titandioxid, Zinkoxid oder Eisenoxiden kombiniert. Als organischer Lichtschutzfilter wird dabei oft Butylmethoxydibenzoylmethan (BMDM) verwendet. Die Wirksamkeit des Butylmethoxydibenzoylmethans wird jedoch in dieser Kombination mit Metalloxiden deutlich reduziert durch die Fähigkeit des Butylmethoxydibenzoylmethans als Diketon mit verschiedenen Metallionen Komplexe zu bilden. Solche Reaktionen sind bekannt mit lonen von Titan, Zink, Eisen und Aluminium. Jedoch müssen die Metallionen nicht primär als lonen vorliegen, sondern können auch als Oxide reagieren. Diese Reaktionen sind in Sonnenschutzformulierungen natürlich nicht erwünscht.

Es bestand daher die Aufgabe, einen Weg zu finden, diese unerwünschte Reaktion zu unterbinden und dadurch eine Verbesserung der Stabilität von kosmetischen Zubereitungen, die Butylmethoxydibenzoylmethan zusammen mit anorganischen Lichtschutzfiltern enthalten, zu erzielen.

Zusammensetzungen die 4-4'-Methoxy-t-butyldibenzoylmethan und ein speziell an die Oberfläche behandeltes Zinkoxid enthalten, sind aus WO 3 215 016 bekannt.

Diese Offenbarung befasst sich mit der Stabilität solcher Zusammensetzungen. Eine Zubereitung, die zusätzlich einen Aldehyd oder ein Ketone enthält, ist aus diesem Dokument nicht bekannt.

Überraschend wurde nun gefunden, daß die unerwünschte Komplexbildung durch anwesende aktive Aldehyde und/oder Ketone unterbunden werden kann. Dabei binden die Aldehyd- und/oder Ketogruppen an die aktiven Stellen der Metalloxidpartikeloberflächen und unterbinden damit die Kontaktmöglichkeit des BMDM an die Metalloxide.

Gegenstand der Erfindung ist somit die Verwendung von Aldehyden und/oder Ketonen zur Stabilisierung von kosmetischen Formulierungen, die Butylmethoxydibenzoylmethan kombiniert mit anorganischen Lichtschutzfiltem enthalten.

Gegenstand der Erfindung ist auch eine kosmetische Zubereitung enthaltend anorganische Lichtschutzfilter sowie Butylmethoxydibenzoylmethan, dadurch gekennzeichnet, daß zur Verbesserung der Stabilität Aldehyde und/oder Ketone zugegeben werden.

Ein weiterer Gegenstand der Erfindung ist ein Verfahren zur Verbesserung der Stabilität von kosmetischen Zubereitungen enthaltend Butylmethoxydibenzoylmethan kombiniert mit anorganischen Lichtschutzfiltem, dadurch gekennzeichnet, daß man Aldehyde und/oder Ketone zusetzt.

Als anorganische Lichtschutzfilter kommen hauptsächlich Titandioxid, Zinkoxid oder Eisenoxide in Frage, femer auch Ceroxid. Femer können diese Oxide auch mit anderen Metalloxiden, beispielsweise mit Aluminiumoxid, nachbeschichtet sein.

Der Gehalt an diesen anorganischen Partikeln in den erfindungsgemäßen Formulierungen beträgt 0.1 bis 30 Gew. %, vorzugsweise 0.5 bis 10 Gew. %.

Das Butylmethoxydibenzoylmethan ist in der erfindungsgemäßen Zubereitung mit einem Gehalt von 0.1 bis 10 Gew. %, vorzugsweise von 0.5 bis 3 Gew. % vertreten.

Das BMDM kann in den erfindungsgemäßen kosmetischen Formulierungen alleine oder auch in Kombination mit einem oder mehreren UV-Filtern anderer Substanzklassen vorliegen, die jeweils in einer Menge von 0.01 bis 40 Gew. %, vorzugsweise von 0.1 bis 10 Gew. % enthalten sein können.

Als Aldehyde oder Ketone kommen sogenannte aktive oder aktivierte Verbindungen in Frage. Vorzugsweise werden Ketole, Diketone oder Ketone oder Aldehyde, die in Konjugation zu einer Doppelbindung stehen, eingesetzt, femer auch Formaldehyd oder formaldehydabspaltende Substanzen.

Beispielhaft seien folgende, besonders bevorzugten Verbindungen genannt: Formaldehyd (auch als formaldehydabspaltendes Konservierungsmittel), Dihydroxyaceton, Vanillin, Erythrulose, Hydroxyaceton, Zucker wie Sorbose, Fructose oder Glucose.

Der Gehalt an Aldehyde und/oder Ketonen beträgt zumindest 100 ppm und höchstens 10 Gew. %.

Es können auch Kombinationen von Aldehyden und Ketonen eingesetzt werden. Dabei kommen vorzugsweise 1:1 Kombinationen in Frage. Beispielsweise eine Kombination von Formaldehyd und Vanillin oder von Formaldehyd und Erythrulose.

Eine ganz besonders bevorzugte erfindungsgemäße Formulierung enthält als Aldehyd Formaldehyd, da dieses der Einfachheit halber gleichzeitig als Konservierungsmittel eingesetzt werden kann.

Die Substanz Butylmethoxybenzoylmethan (Eusolex® 9020) ist, wie schon erwähnt, bekannt und kann im Handel bezogen werden, z.B. von der Firma Merck KGaA, Darmstadt.

Die Aldehyde und Ketone (oder auch deren Vorstufen) können einerseits direkt der kosmetischen Formulierung zugesetzt werden, andererseits ist es auch möglich, die Metalloxide bereits bei ihrer Herstellung, bevor sie der kosmetischen Formulierung zugesetzt werden, mit einem Aldehyd oder Keton zu versetzen oder zu waschen.

Für den erfindungsgemäßen Effekt spielt das keine Rolle.

Gegenstand der Erfindung ist femer ein Verfahren zum Schutz der Haut und/oder natürlicher oder sensibilisierter Haare vor Sonnenstrahlen, wobei auf die Haut oder die Haare eine kosmetische Zubereitung gemäß der vorliegenden Erfindung aufgetragen wird.

Mit "sensibilisierten Haaren" sind Haare gemeint, welche einer Dauerwellenbehandlung, einem Färbe- oder Entfärbeprozeß unterzogen worden sind.

Wird die erfindungsgemäße kosmetische Zubereitung zum Schutz menschlicher Epidermis gegen UV-Strahlen verwendet, liegt sie in verschiedenen, für diesen Typ üblicherweise verwendeten Formen vor. So kann sie insbesondere in Form öliger, ölig-wäßriger, wäßrigalkoholischer oder ölig-alkoholischer Lotionen, Emulsionen, wie als Creme oder als Milch (W/O oder O/W, wobei W = Wasser und O =

Öl bedeutet), in Form ölig-alkoholischer, ölig-wäßriger oder wäßrigalkoholischer Gele oder als feste Stifte oder Puder vorliegen oder als Spray oder Aerosol konfektioniert sein.

Die erfindungsgemäße Formulierung kann weitere kosmetische Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer BMDM, gegebenenfalls mit anderen organischen Lichtschutzfaktoren kombiniert, den anorganischen Lichtschutzfiltem und der erfindungsgemäßen Menge an Aldehyden und/oder Ketonen Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die erfindungsgemäße kosmetische Zubereitung kann auch als alkoholisches Gel vorliegen, welches einen oder mehrere Niedrigalkohole oder-polyole, wie Ethanol, Propylenglycol oder Glycerin, und ein Verdickungsmittel, wie Kieselerde, umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpem.

Ist eine Zubereitung als Aerosol konfektioniert, verwendet man in der Regel die üblichen Treibmittel, wie Alkane, Fluoralkane und Chlorfluoralkane.

Ist eine Formulierung als Spray konfektioniert, verwendet man in der Regel wäßrig-alkoholische Lösungen.

Soll das erfindungsgemäße Mittel natürliche oder sensibilisierte Haare vor UV-Strahlen schützen, so kann es als Shampoo, Lotion, Mousse, Gel oder Emulsion zum Ausspülen vorliegen, wobei die jeweilige Formulierung vor oder nach dem Schampoonieren, vor oder nach dem Färben oder Entfärben, vor oder nach der Dauerwelle aufgetragen wird; oder das Mittel liegt als Lotion, Mousse oder Gel zum Frisieren und Behandeln, als Lotion, Mousse oder Gel zum Bürsten oder Legen einer Wasserwelle, als Haarlack, Dauerwellmittel, Färbeoder Entfärbemittel der Haare vor. Dieses Mittel kann außer der erfindungsgemäßen Verbindung verschiedene, in diesem Mitteltyp verwendete Adjuvanzien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen kosmetischen Zubereitungen können mit Hilfe von Techniken hergestellt werden, die dem Fachmann wohl bekannt sind.

Die erfindungsgemäßen Formulierungen können auch zur vorbeugenden Behandlung von Entzündungen und Allergien der Haut und zur Verhütung bestimmter Krebsarten verwendet werden.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die folgenden Beispiele sollen die Erfindung näher erläutern. Sie sind jedoch nicht dazu geeignet den Gegenstand der Erfindung allein auf diese Beispiele zu beschränken.

### Beispiel 1

Aus folgenden Komponenten stellt man eine erfindungsgemäße Sonnenschutzcreme (O/W) her.

| | | | Gew. % |
|---|---|---|---|
| A | Eusolex® 9020 (Art.-Nr. 105844) | (1) | 1.00 |
| | | | |
| | (Butylmethoxydibenzoylmethan) | | |
| | Arlatone 983 S | (2) | 1.50 |
| | Arlatone 985 | (2) | 2.20 |
| | Brij 76 | (2) | 1.50 |
| | Miglyol 812 Neutralöl | (3) | 10.00 |
| | | | |
| B | Sorbitol F flüssig (Art.-Nr. 102993) | (1) | 2.50 |
| | Propandiol-1,2 (Art.-Nr. 107478) | (1) | 2.50 |
| | Konservierungsstoffe | (1) | q.s. |
| | demin. Wasser | | ad 100.00 |
| | | | |
| C | Carbomer 934 | (4) | 0.50 |
| | | | |
| D | Tris(hydroxymethyl)aminomethan | | |
| | | | |
| | (Art.-Nr. 108386) | (1) | 0.36 |
| | demin. Wasser | | 9.64 |
| | | | |
| E | Eusolex® T-2000 (Art.-Nr. 105373) | (1) | 5.00 |
| | | | |
| | Formaldehyd-Lösung (Art.-Nr. 159174) | (1) | 0.10 |

### Herstellung:

Phase E wird gemischt und sorgfältig gerührt und getrocknet. Man mischt Phase B, gibt das Carbomer 934 hinzu und lässt die Mischung aufquellen, bis sie homogen ist. Danach gibt man die vorgemischte Phase D hinzu und homogenisiert. Nun wird Phase E zu dieser Mischung gegeben und man erwärmt nun auf 80 °C. Die Phase A wird vereinigt und auf 75 °C erwärmt. Unter langsamen Rühren wird Phase A in die vorher aus B-E hergestellte Phase eingerührt.

### Konservierungsstoffe:

0.05% Propyl-4-hydroxybenzoat (Art.-Nr. 107427)
0.15% Methyl-4-hydroxybenzoat (Art.-Nr. 106757)

### Bezugsquellen:

(1) Merck KGaA, Darmstadt
(2) ICI, Essen
(3) Hüls Troisdorf AG, Witten
(4) Goodrich, Neuss

### Beispiel 2

Aus folgenden Komponenten stellt man eine erfindungsgemäße Sonnenschutzcreme (O/W) her.

| | | | Gew. % |
|---|---|---|---|
| A | Eusolex® 9020 (Art.-Nr. 105844) (Butylmethoxydibenzoylmethan) | (1) | 1.00 |
| | | | |
| | Eusolex® T-2000 (Art.-Nr. 105373) | (1) | 5.00 |
| | Arlatone 983 S | (2) | 1.50 |
| | Arlatone 985 | (2) | 2.20 |
| | Brij 76 | (2) | 1.50 |
| | Miglyol 812 Neutralöl | (3) | 10.00 |
| | | | |
| B | Sorbitol F flüssig (Art.-Nr. 102993) | (1) | 2.50 |
| | Propandiol-1,2 (Art.-Nr. 107478) | (1) | 2.50 |
| | Formaldehyd-Lösung (Art.-Nr. 159174) (1) | | q.s. |
| | demin. Wasser | | ad 100.00 |
| | | | |
| C | Carbomer 934 | (4) | 0.50 |
| | | | |
| D | Tris(hydroxymethyl)aminomethan | | |
| | (Art.-Nr. 108386) | (1) | 0.36 |
| | demin. Wasser | | 9.64 |

### Herstellung:

Man mischt Phase B, gibt das Carbomer 934 hinzu und lässt die Mischung aufquellen, bis sie homogen ist. Danach gibt man die vorgemischte Phase D hinzu und homogenisiert. Man erwärmt nun auf 80 °C. Die Phase A wird vereinigt und auf 75 °C erwärmt. Unter langsamen Rühren wird Phase A in die vorher aus B-D hergestellte Phase eingerührt.

### Bezugsquellen:

(1) Merck KGaA, Darmstadt
(2) ICI, Essen
(3) Hüls Troisdorf AG, Witten
(4) Goodrich, Neuss

## Patentansprüche

1. Kosmetische Zubereitung enthaltend anorganische Lichtschutzfilter sowie Butylmethoxydibenzoylmethan, **dadurch gekennzeichnet, daß** zur Verbesserung der Stabilität Aldehyde und/oder Ketone zugegeben werden.

2. Kosmetische Zubereitung nach Anspruch 1, **dadurch gekennzeichnet, daß** als anorganische Lichtschutzfilter Titandioxid, Zinkoxid oder Eisenoxide enthalten sind, wobei diese auch mit anderen Metalloxiden nachbeschichtet sein können.

3. Kosmetische Zubereitung gemäß Anspruch 1 -2, **dadurch gekennzeichnet, daß** als Aldehyde oder Ketone vorzugsweise Ketole, Diketone oder solche Ketone oder Aldehyde, die in Konjugation zu einer Doppelbindung stehen, Formaldehyd oder formaldehydabspaltende Substanzen enthalten sind.

4. Kosmetische Zubereitung nach einem oder mehreren der Ansprüche 1 bis 3, **dadurch gekennzeichnet, daß** der Gehalt an Butylmethoxydibenzoylmethan 0.1 bis 10 Gew. % beträgt.

5. Kosmetische Zubereitung nach einem oder mehreren der Ansprüche 1 bis 4, **dadurch gekennzeichnet, daß** der Gehalt an Aldehyden und/oder Ketonen 100 ppm bis 10 Gew.-% beträgt.

6. Kosmetische Zubereitung nach einem der Ansprüche 1 bis 5, **dadurch gekennzeichnet, daß** der Gehalt an anorganischen Lichtschutzfiltem 0.1 bis 30 Gew. % beträgt.

7. Verwendung von Aldehyden und Ketonen zur Herstellung von Kosmetischen Zubereitungen Gemäß einem oder mehreren der Ansprüche 1 bis 6.

## Claims

1. Cosmetic preparation comprising inorganic light protection filters and butylmethoxydibenzoylmethane, **characterized in that** aldehydes and/or ketones are added to improve the stability.

2. Cosmetic preparation according to Claim 1,
**characterized in that** the inorganic light protection filters present are titanium dioxide, zinc oxide or iron oxides, it also being possible for these to be aftercoated with other metal oxides.

3. Cosmetic preparation according to Claim 1-2, **characterized in that** the aldehydes or ketones present are preferably ketols, diketones or those ketones or aldehydes which are in conjugation to a double bond, formaldehyde or formaldehyde-releasing substances.

4. Cosmetic preparation according to one or more of Claims 1 to 3, **characterized in that** the content of butylmethoxydibenzoylmethane is 0.1 to 10% by weight.

5. Cosmetic preparation according to one or more of Claims 1 to 4, **characterized in that** the content of aldehydes and/or ketones is 100 ppm to 10% by weight.

6. Cosmetic preparation according to one of Claims 1 to 5, **characterized in that** the content of inorganic light protection filters is 0.1 to 30% by weight.

7. Use of aldehydes and ketones for the preparation of cosmetic preparations according to one or more of Claims 1 to 6.

## Revendications

1. Préparation cosmétique contenant des filtres photoprotecteurs minéraux ainsi que du butylméthoxydibenzoylméthane, **caractérisée en ce qu'**on ajoute des aldéhydes et/ou des cétones afin d'améliorer la stabilité.

2. Préparation cosmétique selon la revendication 1, **caractérisée en ce qu'**elle contient comme filtres photoprotecteurs minéraux du dioxyde de titane, de l'oxyde de zinc ou des oxydes de fer, ceux-ci pouvant également être recouverts avec d'autres oxydes métalliques.

3. Préparation cosmétique selon la revendication 1-2, **caractérisée en ce qu'**elle contient en tant qu'aldéhydes ou cétones, de préférence, des cétols, des dicétones ou des cétones ou aldéhydes qui se trouvent en conjugaison par rapport à une double liaison, du formaldéhyde ou des substances libérant du formaldéhyde.

4. Préparation cosmétique selon une ou plusieurs des revendications 1 à 3, **caractérisée en ce que** la teneur en butylméthoxydibenzoylméthane va de 0,1 à 10 % en poids.

5. Préparation cosmétique selon une ou plusieurs des revendications 1 à 4, **caractérisée en ce que** la teneur en aldéhydes et/ou cétones va de 100 ppm à 10 % en poids.

6. Préparation cosmétique selon une ou plusieurs des revendications 1 à 5, **caractérisée en ce que** la teneur en filtres photoprotecteurs va de 0,1 à 30 % en poids.

7. Utilisation d'aldéhydes et de cétones pour la fabrication de préparations cosmétiques selon une ou plusieurs des revendications 1 à 6.
